# EUROPEAN PATENT SPECIFICATION

(11) **EP 0 979 060 B1**
(45) Date of publication and mention of the grant of the patent: **03.08.2005**
(21) Application number: 98911542.3
(22) Date of filing: 10.03.1998
(51) Int. Cl.: A61F 7/00

(54) **IMPROVED APPARATUS FOR THE CORE BODY WARMING OF MAMMALS EXPERIENCING HYPOTHERMIA**
VERBESSERTE VORRICHTUNG ZUR KÖRPERERWÄRMUNG VON SAUGETIEREN UND MENSCHEN DIE AN UNTERKÜHLUNG LEIDEN
APPAREIL AMELIORE POUR LE RECHAUFFEMENT PROFOND DU CORPS DE MAMMIFERES SOUFFRANT D'HYPOTHERMIE

(30) Priority: 10.03.1997 US 813277
(43) Date of publication of application: 16.02.2000
(73) Proprietor: Aquarius Medical Corporation, Scottsdale, Arizona 95260 (US)
(72) Inventor: GRAHN, Dennis, A., Palo Alto, CA 94306 (US); CHANDLER, W., Jeffrey, Phoenix, AZ 85018 (US); KANE, John, R., Scottsdale, AZ 85260 (US)
(74) Representative: Casey, Lindsay Joseph
(86) International application number: PCT/US1998/004648
(87) International publication number: WO 1998/040039

(56) References cited:
- WO-A-96/28120
- FR-A- 2 544 202
- US-A- 4 648 392
- US-A- 5 074 285
- US-A- 5 241 958
- US-A- 5 314 455
- US-A- 5 441 477
- US-A- 5 476 490
- US-A- 5 683 438

## Description

### Field of the Invention

The present invention generally relates to an apparatus for core body warming of hypothermic mammals (hypothermy).

### Background of the Invention

There has recently been tremendous interest in the manipulation of core body temperature in mammals.

Hyperthermia is defined as the condition of a temperature regulator when core body temperature is above its set range specified for the normal active state of the species. Hypothermia is defined as the condition of a temperature regulator when core temperature is below its set range specified for the normal active state of the species. Normothermia is the normal active state of a species, and is defined as the condition of a temperature regulator when its core temperature is within one standard deviation of the range associated with the normal post-absorptive resting condition of the species in a thermoneutral environment or in a range of ambient temperatures at which temperature regulation is achieved only by control of sensible heat loss.

Mammals, such as humans, are characterized by a relatively constant and high core body temperature (above about 37°C in humans). The maintenance of body temperature normally includes heat dissipation to the outside atmosphere through specific heat exchange regions of the body. These regions include (but are not limited to) the faces, ears, and other specific exposed surface areas of the appendages such as hands and feet in a human, the tail in a rodent, and the ears in a mammal such as an elephant or rabbit. The thermal core of the body can be defined as the inner tissues of the body whose temperatures are not changed by circulatory adjustments and changes in heat dissipation to the environment. Under normal conditions, the thermal core includes the heart, brain, lungs and the viscera. The essential thermal core area comprises the heart and the brain. These tissues are distinguishable from the thermal shell of the body that surrounds the thermal core. The thermal shell would insulate the thermal core, unless there is an active heat exchange nexus between the two, such as would occur when there is vasodilation in certain heat transfer regions of the thermal shell.

When the thermoregulatory system is active, hyperthermia involves the inability of the system to adequately respond to a heat challenge with increased heat dissipation. Likewise, hypothermia involves the inability of the system to adequately respond to a cold challenge with increased heat conservation or production.

Regarding hyperthermia, hyperthermia may result from exposure to heat (heat stroke). There is a need for a field portable unit to treat hyperthermia victims that is portable, self-powered, lightweight and non-invasive.

Furthermore, it has been recently reported that perioperative normothermia reduces the incidence of surgical-wound infections and shortens hospitalization stays (Kurz et al., 334 New Eng. J.Med. 1209 (1996)). An apparatus and method to induce and maintain normothermia is thus desired in a hospital setting.

International Patent Specification No. WO-A-96/28120 discloses an apparatus and a method for core body warming of hypothermic mammals.

The present invention is primarily directed to an apparatus for the treatment of hypothermia, whether induced or not, through the use of a non-invasive, portable apparatus.

### Summary of the Invention

According to an aspect of the present invention, there is provided a device as specified in claim 1.

The invention, which is a portable unit, is a device for treating hypothermia in a mammalian body non-invasively, using an apparatus that is portable, lightweight, and requires no external electrical power source. The portable device of the invention may be used by operating rooms, Patient Anesthesia Care Units, emergency rooms and emergency medical treatment personnel under any of the conditions typically experienced in field and hospital settings.

For hypothermia applications, the present invention creates a thermal pipeline directly from the skin surface to the body core, by introducing the skin surface to the simultaneous effects of negative pressure and an external heat source. An appendage, which can be an arm, is encased in a chamber where vacuum is applied and an external heat source is activated. Thereupon the appendage of the patient is vasodilated and effective heat transfer can occur much faster than when compared with prior techniques known in the art.

A technique for raising the core body temperature of mammals suffering hypothermia in an non-invasive manner includes forced air convection warming of the tissue of the affected body. Often, forced warm air over the surface of the victim's body is coupled with application of an insulator to trap external heat, as with blankets. Unfortunately, in a hypothermic mammalian body, the surface tissues are vasoconstricted to prevent blood flow to the skin, in a natural evolutionary response of hypothermic mammals to cold, in an attempt to insulate the core body tissues from atmosphere. The present invention employs a new paradigm of heat transfer to a body by relying on the convective properties of the circulatory system of a mammalian body, by inducing dilation in a selected area of the skin, in opposition to the natural vasoconstrictive response of the body to cold, in order to aid in transferring heat from an external heat source to the core body tissues, though blood circulation between the thermal core and dilated area of the thermal shell (e.g., such as surface tissues). As explained herein, this vasodilation of the selected area of the thermal shell (e.g., surface tissues and blood vessels) is achieved through an application of negative pressure to the selected surface tissues.

The present invention has been estimated to achieve in one typical configuration (negative pressure and heat application to a limb) an accelerated rewarming rate of about 8°C/hour, as opposed to the conventional forced air apparatus' rewarming rate of only about 1°C/hour. Thus, forced air warming could take an estimated order of magnitude longer than the techniques of the present invention to increase core body temperature to a normal core body temperature. The human body will attempt to maintain a certain equilibrium core body temperature when reaching the normal core body temperature. Once normathermia is reached, the body can dissipate any excess heat through the other appendages whose surface tissues have become vasodilated. For this reason, the present invention does not have a risk of inducing hyperthermia.

### Brief Description of the Drawings

Figure 1 shows an exploded view of one preferred embodiment of the invention.
Figures 2-3 show cross-sectional views of the Figure 1 embodiment of the invention in operation.
Figure 4 shows a perspective view of another design of the present invention.
Figure 5 shows a cross-section of the gauntlet or sleeve gasket construction of the embodiment of Figure 4.

### Detailed Description of the Preferred Embodiments

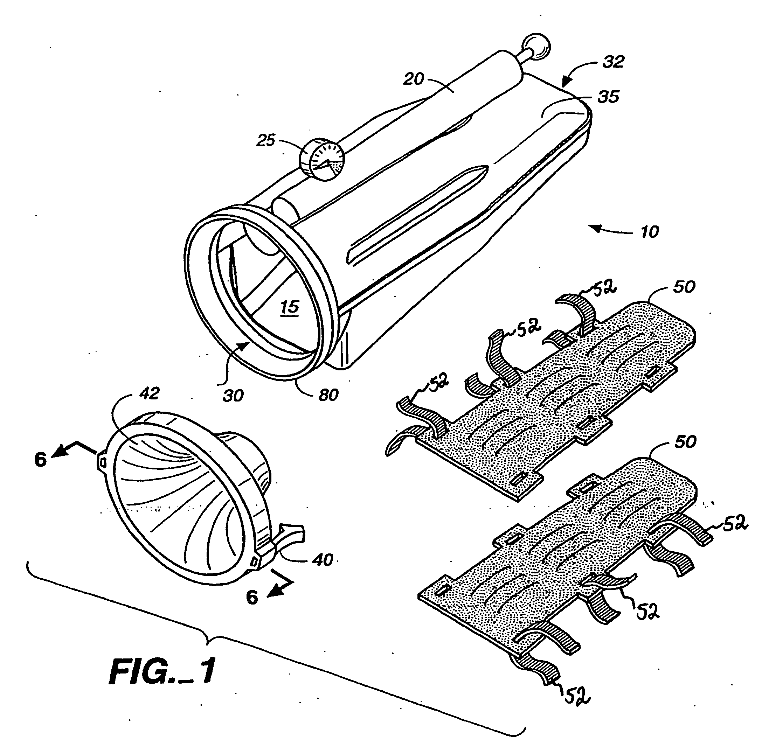

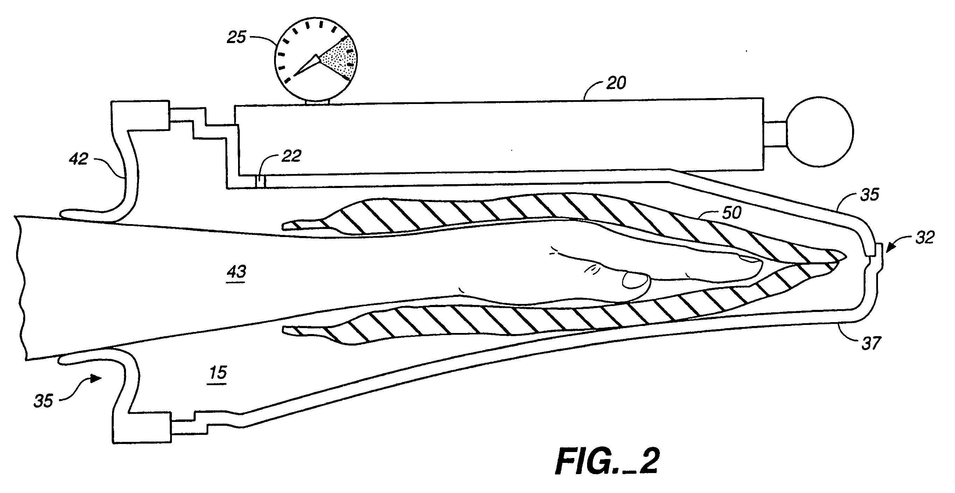

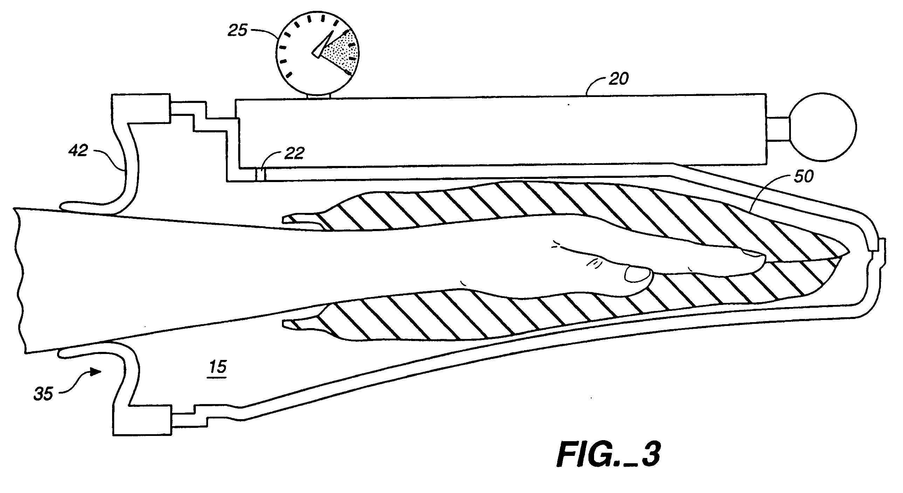

Turning attention to Figures 1-3, there is shown the overall configuration of one embodiment of the apparatus 10 of the present invention, showing in exploded view a vacuum chamber 15, having a vacuum pump 20 with an inlet port 22 to allow the withdrawal of air from inside the vacuum chamber 15, and a gauntlet seal 42 to seal a mammalian forearm 43 inside the vacuum chamber 15. A heating pad 50 envelopes the terminal portion of a mammalian forelimb.

The vacuum pump 20 is preferably a hand pump requiring no external electrical power supply, such as the kind manufactured by Humphrey Products Company. However, other types of means for inducing vacuum may be employed, such as a powered vacuum pump that employs a CO₂ cartridge and venturi valve or other vacuum-inducing means. The vacuum pump 20 may optionally contain either a built-in vacuum pressure gauge 25, or the apparatus itself may have its own separate vacuum gauge. For normal-size human bodies, negative pressures for vasodilation are currently recommended to be in the negative 40-60 mm-Hg range. Higher pressures and external temperatures may result in greater heat transfer. In one study, 150 mm Hg negative pressure was shown to not cause localized tissue damage in healthy adults; however, care should be taken applying very high negative pressures since rupture of blood vessels has been known to occur.

Vacuum chamber 15 has an open mouth end 30 and a closed sealed end 32, with a curvilinear clear top shell 35 and flat opaque bottom shell 37. Preferably the chamber may be made of plastic. At open end 30 of vacuum chamber 15 there is an elastic gasket holder or fastener 40, which may be a clasp as shown, or any other fastener including an O-ring, holding a flexible sleeve or gauntlet 42 that forms one sleeve design for receiving a limb or extremity of a user, such as a human arm 43. Gasket 42 would be deformable to conform to the arm of a user to form a seal and yet be of sufficient strength to withstand a vacuum pressure of at least 300 mm-Hg without bursting, and forms an air seal to avoid leakage from the inside of vacuum chamber 15 to outside atmosphere. In this way, gasket 42 forms a seal in one preferred embodiment of the invention.

Inside vacuum chamber 15 there is found an external heat source comprising a chemically-activated heating pad 50, such as the sodium acetate kind manufactured under the tradename "Proheat™," manufactured by Prism Technologies of San Antonio, Texas, or by Reheater, Inc. of Santa Fe Springs, California, of appropriate sufficient size to cover or substantially cover a normalsized user's hand and forearm or other appendage that is to be treated. Other types of heat sources and designs are contemplated within the scope of the invention, including heating sources using liquid sodium acetate, gel sodium acetate, dry iron oxide pad designs, or water perfusion or electric glove pad designs (with suitable externally-supplied hot water or electric power supplies).

In the preferred embodiment shown in Figures 1-3, the chemically-activated heating pad, when a reagent chemical activator is broken, develops an exothermic chemical reaction that releases heat, and expands the size of the pad. The heating pad 50 may be formed in a double layer design, such as an oven mitt or glove, to receive a hand, forearm or other body extremity inside the heating pad, and may have Velcro straps 52 holding the two pads together as shown in Figure 1; or the pad may be one piece.

operation of the apparatus of Figures 1-3 will now be described. When treating a mammalian victim suffering from hypothermia in the field, an unattached gasket 40 is pulled down an appendage of the victim, such as the bare hand, forearm, arm or foot in the case of a human, or other suitable appendage in the case of another species of mammal. At this point, the gasket is preferably pulled a small distance toward the distal end of the appendage so that the flange 42 inverts its normally inward-facing direction. The appendage is then is enveloped in chemical heating pad 50, and the pad is activated to generate heat. The patient victim's appendage 43 is then inserted by the operator of the apparatus into the vacuum chamber 15 and the gasket 40 is attached to the lid 80 of the vacuum chamber 15. The operator then evacuates the vacuum chamber to form a vacuum by pumping by hand vacuum pump 20 a predetermined number of strokes or until the vacuum gauge 25 shows a range of vacuum of between 40 to 60 mm-Hg. The vacuum will cause the victim's non-core surface and skin tissue of the appendage within the vacuum chamber 15 to vasodilate, so that effective heat transfer may occur from the chemical heating pad through the vasodilated tissue of the appendage to the victim's core body tissue.

As discussed above, apparatus of the present invention is predicted to provide a significant improvement over traditional core body warming techniques. The apparatus of the present invention is estimated to achieve an rewarming rate of about 8°C/hour, as opposed to the conventional forced air apparatus' rewarming rate of only about 1°C/hour. Thus, forced air warming could take an estimated order of magnitude longer than the techniques of the present invention to increase core body temperature to a normal core body temperature.

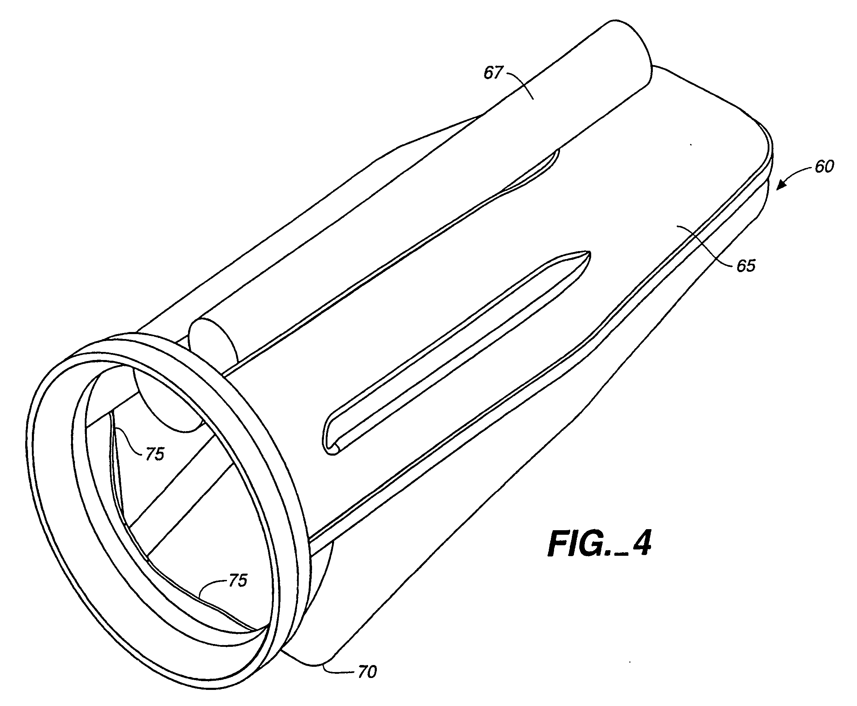

Figure 4 shows another embodiment of the present invention that is very similar to the embodiment of Figures 1-3. The hypothermia apparatus or device 60 has a plastic housing 65, which may be of a unitary construction or a modular two-piece construction, with or without a clear plastic top half, and having a recessed portion to house a pump 67, which may be of the same construction as the hand pump 20 of the Figure 1 embodiment, to achieve a compact design. The pump would have suitable openings leading to the inside (vacuum chamber 69) of the apparatus 60, and to atmosphere, so that the vacuum chamber may be evacuated of air. The pump may be powered to operate through non-manual, externally powered means as well. The housing 65 has a flat surface rectangular base 70 that enables the device to be laid flat on a table. A seal or gasket 75 is removably fastened to the lip or lid 80 of the housing 65 of the device through suitable fasteners, such as clamps or clasps (not shown) which may be any suitable fastener to hold an annular elastomer containing an opening for passage of a forearm in place around the open container end of apparatus 60. As in the prior embodiment, a suitable appendage such as a hand and/or forearm of a mammalian user would be placed into the inside of the housing (the vacuum chamber 69), which is hollow to receive the appendage, and the gasket 75 would form a substantially airtight chamber from which air may be pumped out by pump 67 and heat may be applied by an external heating source such as an exothermic chemical heat pack, as previously described. The dimensions of the apparatus are about the length of an average human forearm to the elbow, or about 18 inches (46 cm). The weight of the device is about two pounds.

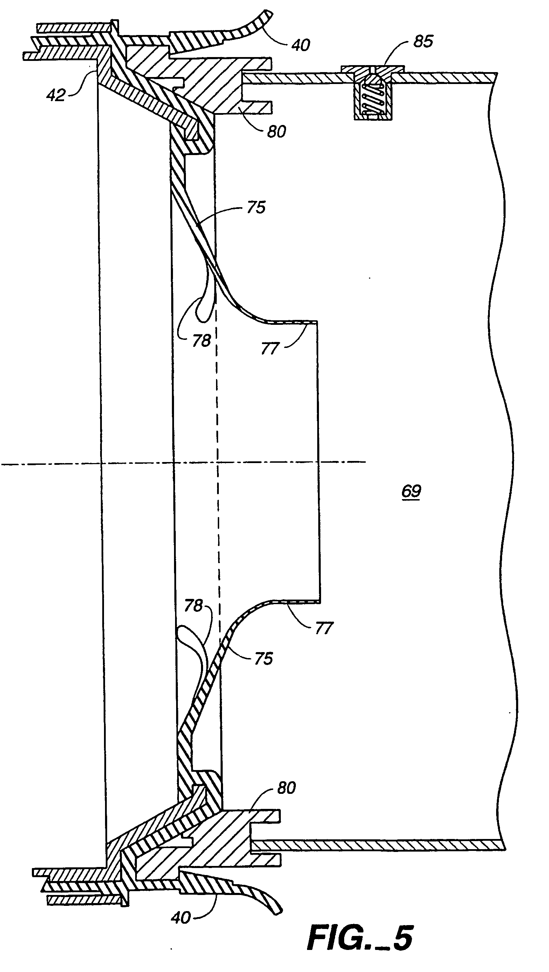

Turning to Figure 5, there is shown the gasket gauntlet 75 which forms the seal for the hypothermia apparatus, similar to the Figure 1 embodiment, by sealing against the user's forearm (not shown) when the forearm is placed into the vacuum chamber. The gasket, being annular and having a small-sized annular opening sufficient to snugly receive an average user's appendage, is preferably made of a elastomeric material such as natural or artificial rubber, latex or an elastomer such as "Dynaflex™," manufactured by Kraton Polymers, having a Shore hardness of 20-30 or "Clopay™" material. The gasket 75 has a thin tapered portion 77 that initially extends into the hollow vacuum chamber 69 of the apparatus 60, and a thicker lip portion that is suitably removably but fixedly engaged to the lid 80 of the device. Dimensions of the seal are approximately about 6 inches (15 cm) wide at the mouth and 2 inches (5 cm) at the narrow portion receiving the arm of a user. To assist personnel in breaking the vacuum once treatment is commenced when a user's appendage is inside the vacuum chamber 69, one or more pull tabs 78 may be placed on the gasket 75, which would be grasped to pull away the gasket 75 from the forearm to allow air into vacuum chamber 69 and thus break the vacuum, thereby forming a manual relief valve. Other spring-biased or automatic relief valves, such as relief valve 85, venting the vacuum chamber to atmosphere may be provided in the apparatus of the present invention to prevent excessive negative atmosphere from forming inside.

As before, the operation of the apparatus when treating hypothermy would consist of a mammalian user having a suitable appendage such as a hand or forearm inserted into the hollow chamber of the device. The thin tapered portion 77 of the seal would conform around the appendage. The gasket can be seated against a limb further by pulling the appendage a bit out of the apparatus (away from the inside of the apparatus) to cause the thin flexible portion 77 to invert its normally inward-facing direction. In any event, a substantially airtight seal is formed between the user's forearm and the gasket inside the vacuum chamber of the apparatus, and heat from an external heat source is applied around the portion of the arm inside the vacuum chamber. Previously, the chemical heat source had been wrapped around the hand of the user prior to the forearm being inserted into the device. Vacuum is then applied to evacuate the inside vacuum chamber of the apparatus. The combination of vacuum formed and heat applied would be sufficient to begin the process of vasodilation. Typically, for a human being, the necessary vacuum would occur in the range of between 40-60 mm-Hg vacuum. The device may include an optional external port for application of an externally-operated vacuum generating device such as an electrically powered, non-manual vacuum pump. In the preferred embodiment shown, which is suitable for field use, a manual mechanical hand pump supplies sufficient vacuum to the vacuum chamber. Upon achieving of the adequate vacuum in vacuum chamber 69, which may be monitored by a pressure gauge (not shown), and upon the application of external heat to the appendage inside the apparatus, an increase in core body temperature should occur.

Further, in the operation of the present invention, one contemplated preferred setting for use of the hypothermy apparatus is in a field setting, at the site where a mammal patient is suffering from hypothermia, and outside a clinical setting (hospital). Thus the present invention, being portable, is particularly well adapted for use by Emergency Medical Treatment personnel.

Other preferred specifications for either preferred embodiment of the hypothermia apparatus of the present invention are as follows:

### Construction:

-- the housing body or sleeve body should be made of a substantially clear or transparent plastic material in at least half of its body to enable a patient's arm and hand to be seen when inside the device;
-- the plastic material must be durable and the housing not break if dropped from a height of 30 inches onto a vinyl tile surface;
-- the housing should not have sharp edges;
-- the housing must be able to withstand a vacuum of at least 300 mm Hg;
-- the housing must operate properly at ambient temperatures from -18°C to 70°C;
-- all materials forming the hypothermia apparatus be non-allergenic, and capable of withstanding cleaning with alcohol and other cleaning reagents used in a hospital environment;
-- the longitudinal length of the housing must not exceed 46 cm (18 inches), the lateral width must not exceed 25.4 cm (10 inches), the height must not exceed 20.3 cm (8 inches), and the vacuum chamber must be able to accept a forearm that is 46 cm (18 inches) in circumference;
-- the maximum weight of the apparatus not to exceed 5 pounds.

### Operating Features:

-- The vacuum chamber, when receiving a forearm sealed by a gasket, such as gasket 75, should be able to achieve a 50 mm Hg vacuum with not more than eight pumps of a hand operated vacuum pump;
-- the gasket when properly seated on a patient's arm should maintain the required vacuum for thirty minutes without having to use the pump;
-- a relief valve must limit the maximum vacuum to 80 mm-Hg; a relief valve must also be a manual relief valve that will relieve vacuum to permit the removing of the unit from the patient's arm;
-- a vacuum gauge must be present with a color-coded "green zone" indicating a proper operating range;
-- the apparatus must have additional straps or fasteners for securing the apparatus to limit the ability of a patient to move their arm about with the apparatus attached.

### Chemical Activated Heating Pad:

-- The heat range of the chemically activated heating pad when activated in an ambient temperature of 21.1°C or 70°F, will achieve a maximum temperature of about 46°C-47°C or about 115°F - 117°F;
-- the chemically activated heating pad should maintain a temperature within about 5°F of the maximum rated temperature for about 60 minutes in the evacuated vacuum chamber;
-- the pad will be designed to apply heat to both sides of a hand and to the forearm.

Although the present invention is primarily directed to the treatment of hypothermia, it is envisioned that with suitable modification the invention has numerous other applications. These applications concern the manipulation of the core body temperature, for example, in the treatment of hyperthermia.

One example concerns the active manipulation of core body temperature. Feedback means could be employed to monitor the temperature of a patient subject who would have one or more limbs enclosed by the apparatus of the present invention. The hypothermia treating apparatus, described above, can be employed to raise the temperature of the patient whenever the temperature monitoring means indicates that the core body temperature of the patient is lower than desired. A hyperthermia treating apparatus can be employed to lower the temperature of the patient whenever the temperature monitoring means indicates that the core body temperature of the patient is higher than desired.

Although several preferred embodiments of this invention have been described in detail herein with reference to the accompanying drawings, it is to be understood that the invention is not limited to these precise embodiments, and that various changes and modifications may be effected therein by one skilled in the art. Accordingly it is to be understood that while the invention has been described above in conjunction with preferred specific embodiments, the description and examples are intended to illustrate and not limit the scope of the invention, which is defined by the appended claims.

## Claims

1. A portable device (60) for treating hypothermia in a mammal, comprising: a housing (65), the housing (65) having an open end, a closed end and hollow to form a chamber (69), the chamber of sufficient size to receive an appendage (43) from a mammal patient; a pump (67) attached to the housing (65), the pump (67) operatively connected to the chamber to produce a negative atmosphere in the chamber (69); a heating pad (50) to produce heat for the appendage (43); wherein the portable device (60) may be used to treat hypothermia through the application of heat and negative atmosphere on the appendage (43) of the mammal patient;
**characterised in that** the device (60) further comprises a relief valve (85) to vent the vacuum chamber to atmosphere.

2. A device (60) as claimed in Claim 1, further comprising: an annular gasket (75) disposed about the open end of the housing (65), the annular gasket (75) having an opening of smaller diameter than the open end of the housing (65), the annular gasket opening sized to receive the appendage (43) from the mammal patient.

3. A device (60) as claimed in Claim 2, wherein the annular gasket (75) is removably secured to the open end of the housing (65), is of sufficient length to extend into the chamber of the housing (65), and the relief valve comprises a pull tab (78) on a portion of the gasket (75) facing outside the chamber (69) to allow an operator to pull on the gasket (75), and wherein the gasket (75) at the open end receives the appendage (43), to form a substantially air-tight seal with the appendage (43) to seal the chamber (69) from atmosphere to allow vacuum to be formed in said chamber (69), wherein the vacuum may be broken by pulling on the pull tab (78) to release the gasket (75) from the appendage.

4. A device (60) as claimed in Claim 2 or Claim 3, wherein the annular gasket (75) is made of an elastomer selected from the group consisting of natural rubber, artificial rubber, latex, styrene block copolymeric thermoplastic elastomer or styrenic elastomer.

5. A device (60) as claimed in any of Claims 1-3, wherein the heating pad (50) is a chemical heating pad, the pad producing a thermochemical exothermic reaction, and the pad sized to fit around the appendage (43) and, preferably, wherein the chemical heating pad (50) is comprised of material to produce the exothermic reaction that is selected from the group consisting of liquid sodium acetate, or gel sodium acetate.

6. A device as claimed in Claim 4, wherein the heating pad (50) when activated produces a temperature of up to 44.5°C (112°F) from an ambient temperature of 21°C (70°F).

7. A device as claimed in any of Claims 1-5, where the housing (65) is formed of a substantially transparent material, to allow visual inspection of the chamber (69), and, preferably, wherein the housing (65) has a longitudinal dimension of about 460mm (1.8 inches) and a weight of about 907g (two pounds).

## Patentansprüche

1. Tragbare Vorrichtung (60) zum Behandeln von Hypothermie bei einem Säugetier, umfassend: ein Gehäuse (65), wobei das Gehäuse (65) ein offenes Ende, ein geschlossenes Ende und einen Hohlraum zum Bilden einer Kammer (69) aufweist, wobei die Kammer ausreichend Größ zum Aufnehmen eines Anhangs (43) von einem Säugetierpatienten ist; eine an dem Gehäuse (65) befestigte Pumpe (67), wobei die Pumpe (67) operativ mit der Kammer verbunden ist, um eine negative Atmosphäre in der Kammer (69) zu erzeugen; ein Heizkissen (50) zum Erzeugen von Wärme für den Anhang (43); wobei die tragbare Vorrichtung (60) zum Behandeln von Hypothennie durch die Anwendung von Wärme und negativer Atmosphäre auf den Anhang (43) des Säugetierpatienten verwendet werden kann;
**dadurch gekennzeichnet, dass** die Vorrichtung (60) ferner ein Entlastungsventil (85) zum Entlüften der Vakuumkammer an die Atmosphäre aufweist.

2. Vorrichtung (60) nach Anspruch 1, ferner umfassend: ein Ringdichtung (75), die um das offene Ende der Gehäuses (65) herum angeordnet ist, wobei die Ringdichtmig (75) eine Öffnung mit kleinerem Durchmesser als das offene Ende des Gehäuses (65) aufweist, und die Ringdichtungsöfihung Abmessungen zum Aufnehmen des Anhangs (43) von dem Säugetierpatienten aufweist.

3. Vorrichtung (60) nach Anspruch 2, bei der die Ringdichtung (75) entfernbar an dem offenen Ende des Gehäuses (65) befestigt ist, eine ausreichende Länge hat, um sich in die Kammer des Gehäuses (65) auszustrecken, und das Entlastungsventil einen Zuglappen (78) an einem Teil der Dichtung (75) aufweist, der zur Außenseite der Kammer (69) gerichtet ist, um einer Bedienungsperson Ziehen an der Dichtung (75) zu ermöglichen, und wobei die Dichtung (75) an dem offenen Ende den Anhang (43) aufnimmt, um eine im wesentlichen luftdichte Dichtung mit dem Anhang (43) zum Abdichten der Kammer (69) von der Atmosphäre zu bilden, damit ein Vakuum in der genannten Kammer (69) gebildet werden kann, wobei das Vakuum durch Ziehen an dem Zuglappen (78) zum Lösen der Dichtung (75) von dem Anhang aufgelöst werden kann.

4. Vorrichtung (60) nach Anspruch 2 oder Anspruch 3, bei der die Ringdichtung (75) aus einem Elastomer besteht, das aus der Gruppe ausgewählt wird, die aus Naturgummi, Kunstgummi, Latex, thermoplastischen Styrolblockcopolymer-Elastomer, oder Styrolelastomer besteht.

5. Vorrichtung (60) nach einem der Ansprüche 1-3, bei der das Heizkissen (50) ein chemisches Heizkissen ist, wobei das Kissen eine thermochemische wärmeliefernde Reaktion erzeugt, und das Kissen Abmessungen aufweist, um um den Ansatz (43) zu passen, und wobei das chemische Heizkissen (50) vorzugsweise ein Material zum Erzeugen der wärmeliefernden Reaktion aufweist, das aus der aus flüssigem Natriumacetat oder Gelnatriumacetat bestehenden Gruppe ausgewählt ist.

6. Vorrichtung nach Anspruch 4, bei der das Heizkissen (50) bei Aktivierung eine Temperatur von bis zu 44,5°C (112°F) von einer Umgebungstemperatur von 21°C (70°F) erzeugt.

7. Vorrichtung nach einem der Ansprüche 1-5, bei der das Gehäuse (65) aus einem im wesentlichen durchsichtigen Material gebildet wird, um visuelle Prüfung der Kammer (69) zuzulassen, und bei der das Gehäuse (65) vorzugsweise eine Längsabmessung von etwa 460 mm (18 Zoll) und ein Gewicht von etwa 907g (zwei engl. Pfund) aufweist.

## Revendications

1. Dispositif portable (60) servant à traiter l'hypothermie chez un mammifère, comprenant : un logement (65), le logement (65) ayant une extrémité ouverte, une extrémité fermée et étant creux pour former une chambre (69), la chambre ayant une taille suffisante pour recevoir un appendice (43) d'un patient mammifère ; une pompe (67) qui est attachée au logement (65), la pompe (67) étant raccordée de façon opérationnelle à la chambre afin de produire une atmosphère négative dans la chambre (69) ; un patin de chauffage (50) destiné à produire de la chaleur pour l'appendice (43) ; dans lequel le dispositif portable (60) peut être utilisé pour traiter l'hypothermie grâce à l'application de chaleur et d'une atmosphère négative sur l'appendice (43) du patient mammifère ;
**caractérisé en ce que** le dispositif (60) comprend en outre une soupape de détente (85) pour mettre la chambre à dépression à l'atmosphère.

2. Dispositif (60), selon la revendication 1, comprenant en outre : un joint annulaire (75) qui est disposé autour de l'extrémité ouverte du logement (65), le joint annulaire (75) ayant une ouverture dont le diamètre est inférieur à celui de l'extrémité ouverte du logement (65), alors que l'vuverture du joint annulaire est dimensionnée de façon à recevoir l'appendice (43) du patient mammifère.

3. Dispositif (60), selon la revendication 2, dans lequel le joint annulaire (75) est assujetti de façon amovible sur l'extrémité ouverte du logement (65), a une longueur suffisante pour s'étendre jusque dans la chambre du logement (65), et la soupape de détente comporte une languette de traction (78) sur une partie du joint (75) dirigée vers l'extérieur de la chambre (69) pour permettre à un opérateur de tirer sur le joint (75), et dans lequel le joint (75) au niveau de l'extrémité ouverte reçoit l'appendice (43), afin de constituer un joint essentiellement étanche avec l'appendice (43) afin de sceller la chambre (69) par rapport à l'atmosphère et pour permettre la formation du vide dans ladite chambre (69), dans lequel il est possible d'éliminer le vide en tirant sur la languette de traction (78) pour libérer le joint (75) de l'appendice.

4. Dispositif (60), selon la revendication 2 ou la revendication 3, dans lequel le joint annulaire (75) est fabriqué dans un élastomère ayant été sélectionné à partir du groupe composé des matériaux suivants : caoutchouc naturel, caoutchouc artificiel, latex, élastomère thermoplastique copolymérique à blocs de styrène ou élastomère styrénique.

5. Dispositif (60), selon l'une quelconque des revendications 1-3, dans lequel le patin de chauffage (50) est un patin de chauffage chimique, le patin produisant une réaction exothermique thermochimique, alors que le patin est dimensionné pour s'adapter autour de l'appendice (43) et, de préférence, dans lequel le patin de chauffage chimique (50) est constitué d'un matériau destiné à produire la réaction exothermique lequel est sélectionné à partir du groupe composé d'acétate de sodium sous forme de liquide ou d'acétate de sodium sous forme de gel.

6. Dispositif selon la revendication 4, dans lequel le patin de chauffage (50) produit, quand il est activé, une température allant jusqu'à 44,5° C (112° F) à partir d'une température ambiante de 21°C (70° F).

7. Dispositif, selon l'une quelconque des revendications 1-5, dans lequel le logement (65) est fabriqué dans un matériau essentiellement transparent, afin de permettre une inspection visuelle de la chambre (69) et, de préférence, dans lequel le logement (65) a une dimension longitudinale de 460 mm (18 pouces) environ et a un poids de 907 g (deux livres) environ.
